# EUROPÄISCHE PATENTANMELDUNG

(11) **EP 2 415 395 A1**
(43) Veröffentlichungstag der Anmeldung: **08.02.2012**
(21) Anmeldenummer: 10171883.1
(22) Anmeldetag: 04.08.2010
(51) Int. Cl.: A61B 5/00

(54) **Medizinische Vorrichtung mit Aufnahmekanal für Körperflüssigkeit**

(71) Anmelder: Roche Diagnostics GmbH, 68305 Mannheim (DE); F. Hoffmann-La Roche AG, 4070 Basel (CH)
(72) Erfinder: Frey, Stephan-Michael, 64347 Griesheim (DE); Arnold, Marc, 69517 Gorxheimertal (DE); Brugger, Tobias, 71404 Korb (DE); Braun, Jürgen, 71139 Ehningen (DE)
(74) Vertreter: Stößel, Matthias

(57) **Zusammenfassung**

Es wird eine medizinische Vorrichtung (110) zur Durchführung mindestens einer medizinischen Funktion vorgeschlagen. Die medizinische Vorrichtung (110) umfasst mindestens ein auf einer Körperoberfläche eines Benutzers aufbringbares Steuerteil (120) und mindestens ein an mindestens einer Insertionsstelle in ein Körpergewebe des Benutzers insertierbares Funktionselement (114). Das Funktionselement (114) ist mit dem Steuerteil (120) verbindbar. Das Funktionselement (114) ist eingerichtet, um mindestens eine medizinische Funktion durchzuführen. Das Steuerteil (120) weist mindestens ein Basisteil (124) mit mindestens einer der Körperoberfläche zuweisenden Auflagefläche (142) auf. Das Basisteil (124) weist mindestens einen Aufnahmekanal (158) zum Aufnehmen von aus der Insertionsstelle austretender Körperflüssigkeit auf.

## Beschreibung

### Gebiet der Erfindung

Die Erfindung betrifft eine medizinische Vorrichtung zur Durchführung mindestens einer medizinischen Funktion sowie ein Basisteil zum Einsatz in einer derartigen medizinischen Vorrichtung. Derartige medizinische Vorrichtungen und Basisteile werden insbesondere im Bereich der medizinischen Diagnostik und/oder Therapeutik eingesetzt. Beispielsweise kann die medizinische Vorrichtung als Langzeitsensor ausgestaltet sein und mindestens ein in ein Körpergewebe des Benutzers insertierbares Sensorelement und ein mit diesem Sensorelement verbundenes, auf einer Körperoberfläche des Benutzers aufgebrachtes Steuerteil umfassen, um ein oder mehrere Analyte in einer Körperflüssigkeit des Benutzers qualitativ und/oder quantitativ zu erfassen, beispielsweise über einen Zeitraum von mehreren Tagen, Wochen oder Monaten hinweg. Alternativ oder zusätzlich kann die medizinische Vorrichtung auch für therapeutische Zwecke eingerichtet sein und kann beispielsweise als Medikationsvorrichtung ausgestaltet sein. So kann die medizinische Vorrichtung beispielsweise mindestens eine in ein Körpergewebe des Benutzers insertierbare Kanüle sowie mindestens ein mit dieser Kanüle verbundenes, auf der Körperfläche angeordnetes Steuerteil umfassen, beispielsweise mit mindestens einer Medikationspumpe, beispielsweise um ein oder mehrere Medikamente über einen längeren Zeitraum hinweg in das Körpergewebe zu applizieren. Verschiedene andere Ausgestaltungen sind möglich.

### Stand der Technik

Aus dem Stand der Technik sind zahlreiche medizinische Vorrichtungen mit diagnostischen, therapeutischen oder chirurgischen Funktionen bekannt. Insbesondere die Überwachung und/oder Beeinflussung bestimmter Körperfunktionen, vorzugsweise die Überwachung einer oder mehrerer Konzentrationen bestimmter Analyte, spielt bei der Vorbeugung und Behandlung verschiedener Krankheiten wie beispielsweise Diabetes eine wesentliche Rolle. Ohne Beschränkung weiterer möglicher Anwendungen wird die Erfindung im Folgenden im Wesentlichen unter Bezugnahme auf eine Blutglukoseüberwachung beschrieben, insbesondere unter Bezugnahme auf eine kontinuierliche Langzeit-Blutglukoseüberwachung über mehrere Stunden, Tage, Wochen oder sogar Monate. Grundsätzlich ist die Erfindung jedoch auch auf andere Arten von Analytüberwachungen und/oder die Überwachung anderer Arten von Körperfunktionen sowie auf andere Gebiete der Medizin übertragbar. Insbesondere kann die Erfindung auch auf die medizinische Therapeutik angewandt werden, beispielsweise auf Medikationspumpen wie beispielsweise Insulinpumpen.

Neben sogenannten Punktmessungen eines oder mehrerer Analyte, bei welchen einem Benutzer gezielt eine Probe einer Körperflüssigkeit entnommen wird, etablieren sich zunehmend auch kontinuierliche Messungen. So etabliert sich beispielsweise eine kontinuierliche Glukosemessung im Interstitium (auch als continuous monitoring, CM, bezeichnet) in jüngerer Vergangenheit als wichtige Methode zum Management, zur Überwachung und zur Steuerung beispielsweise eines Diabetes-Status. In der Regel beschränkt sich diese kontinuierliche Überwachung vorerst auf Diabetiker vom Typ I, also Diabetiker, welche üblicherweise auch eine Insulinpumpe tragen. Auch andere Benutzertypen kommen jedoch nach und nach in Betracht. Mittlerweile kommen in der Regel direkt implantierte elektrochemische Sensoren zum Einsatz, welche häufig auch als nadelartige Sensoren (needle-type sensors, NTS) bezeichnet werden. Dabei wird der aktive Sensorbereich direkt an den Messort gebracht, welcher in der Regel im interstitiellen Gewebe angeordnet ist, und beispielsweise unter Verwendung eines Enzyms (beispielsweise Glukoseoxidase, GOD) Glukose in elektrische Ladungen umgesetzt, die im Verhältnis zur Glukosekonzentration stehen und als Messgröße verwendet werden können. Beispiele derartiger transkutaner Messsysteme sind in US 6,360,888 B1 oder in US 2008/0242962 A1 beschrieben.

Aktuelle Continuous Monitoring-Systeme sind somit in der Regel transkutane Systeme. Dies bedeutet in der Regel, dass der eigentliche Sensor zumindest teilweise unterhalb der Haut des Benutzers angeordnet ist. Ein Steuerteil des Systems, welches auch als Patch bezeichnet wird, befindet sich jedoch in der Regel außerhalb des Körpers des Benutzers, also außerhalb des menschlichen oder tierischen Körpers. Der Sensor wird dabei in der Regel mittels eines Insertionsbestecks appliziert und durch eine Hautoberfläche in das Körpergewebe insertiert, was exemplarisch ebenfalls in US 6,360,888 B1 beschrieben wird. Auch andere Arten von Insertionsbestecken sind bekannt. Die Tragedauer eines Sensors beträgt in der Regel ca. eine Woche, wobei jedoch auch längere Tragedauern, beispielsweise bis hin zu einem oder mehreren Monaten, möglich sind. Danach lassen in der Regel Einflüsse, wie beispielsweise ein Enzymverbrauch und/oder eine Abkapselung im Körper, die Sensitivität des Sensors abfallen, und damit ist ein Ausfall des Sensors zu erwarten. Die Verlängerung der Tragedauer stellt ein aktuelles Entwicklungsgebiet dar. Dies bedeutet jedoch, dass der Sensor und optional mit ihm unmittelbar in Verbindung stehende Komponenten, wie beispielsweise eine Insertionsnadel, als austauschbare Bauelemente ausgestaltet werden sollten. Dementsprechend stellen der Sensor und in der Regel weitere austauschbare Komponenten der Vorrichtung einen sogenannten Einwegteil (disposable) dar. Eine Ansteuereinheit des Steuerteils, welche teure Komponenten des selben (wie beispielsweise hochohmige Verstärker-Eingangsstufen und/oder Potentiostaten und/oder ähnliche aktive Bauelemente) umfasst, wird jedoch in der Regel wiederverwendet, sodass die Vorrichtung in vielen Fällen mindestens einen Mehrwegteil (Reusable) umfasst.

Bei implantierbaren Sensoren umfasst der Einwegteil in der Regel ein sogenanntes Bodymount, welches auf eine Hautoberfläche des Benutzers fixiert werden kann. Beispielsweise enthält das Bodymount in der Regel ein Basisteil und mindestens ein Pflaster, um dieses Basisteil auf der Hautoberfläche zu fixieren. Mit diesem Bodymount, welches in der Regel auch den insertierbaren Sensor trägt, wird dann das Reusable verbunden, welches die wesentlichen Teile einer Ansteuer- und/oder Auswerteelektronik für die Messung der Analytkonzentration enthalten kann. Im Bodymount selbst können jedoch Komponenten angeordnet werden, wie beispielsweise mindestens eine Batterie, sodass mit dem Austausch eines Bodymounts und dem Zusammenfügen eines neuen Bodymounts mit dem Reusable die Vorrichtung gleichzeitig auch mit einer neuen Energiequelle ausgestaltet wird.

Eine Problematik bei medizinischen Vorrichtungen mit insertierbaren Funktionselementen allgemein besteht dabei darin, dass während oder nach der Insertion aus der Insertionsstelle, an welcher das Funktionselement oder ein Teil des selben die Körperoberfläche durchdringt, Körperflüssigkeit wie beispielsweise Blut oder interstitielle Flüssigkeit, austreten kann. Diese Körperflüssigkeit kann das Funktionselement und/oder andere Bauteile der medizinischen Vorrichtungen kontaminieren. So kann beispielsweise Körperflüssigkeit auf elektrische Kontakte eines Sensorelements gelangen, welche innerhalb des Steuerteils angeordnet sind und dort beispielsweise Leckströme oder andere elektronische Störungen verursachen.

Aus US 5,951,521 ist ein implantierbares subkutanes Set für eine Montage auf eine Hautoberfläche eines Benutzers bekannt. Dabei wird während einer Insertion eines subkutanen Sensors eine Basis mit einer Kanüle verwendet. Die Kanüle weist ein Lumen auf, in welchem der Sensor angeordnet ist. Weiterhin wird vorgeschlagen, eine Schlauchleitung ins Innere der Kanüle in das Lumen hinein zu führen, um Körperflüssigkeit, welche sich in dem Lumen nach der Insertion ansammelt, abzusaugen. Die Schlauchleitung ist mit einem Anschluss versehen, welcher beispielsweise mit einer Spritze verbunden werden kann.

Aus US 2003/0004403 A1 sind Verfahren und Vorrichtungen für eine kontinuierliche Überwachung physiologisch relevanter Körperzustände bekannt. Unter Anderem wird dabei vorgeschlagen, einen sogenannten Biointerface Head (BIH) zu verwenden, welcher in ein Körpergewebe implantiert wird. Unter Anderem wird dabei vorgeschlagen, den Biointerface Head mit einer hohlen Schlauchleitung auszugestalten, über welche beispielsweise entzündungshemmende Medikamente in das Körpergewebe eingeführt oder überschüssige Körperflüssigkeit aus dem Körpergewebe herausgeführt werden kann.

Keine der oben beschriebenen Vorrichtungen löst jedoch die skizzierte Problematik, dass an der Insertionsstelle austretende Körperflüssigkeit die medizinische Vorrichtung kontaminieren kann. Im Gegenteil führen die in beiden genannten Druckschriften gezeigten Schlauchverbindungen sogar dazu, dass zusätzlich aus dem Inneren des Körpergewebes Körperflüssigkeit aus dem Körper des Benutzers herausgeführt werden kann, die dort die medizinische Vorrichtung kontaminieren kann und die dort separat entsorgt werden muss. In beiden Dokumenten wird zudem ein separater Kanal in Verbindung mit einem Sensor oder einer Kanüle beschrieben, welcher zusätzlichen Bauraum benötigt und somit eine Insertion des Sensors erschwert.

### Aufgabe der Erfindung

Es ist dementsprechend eine Aufgabe der vorliegenden Erfindung, eine medizinische Vorrichtung mit mindestens einem in ein Körpergewebe eines Benutzers insertierbaren Funktionselement anzugeben, welche die Nachteile bekannter medizinischer Vorrichtungen vermeidet. Insbesondere soll verhindert werden, dass während oder nach der Insertion austretende Körperflüssigkeit die medizinische Vorrichtung in störender Weise kontaminiert oder die Funktionalität der medizinischen Vorrichtung beeinträchtigt.

### Offenbarung der Erfindung

Diese Aufgabe wird gelöst durch eine medizinische Vorrichtung und ein Basisteil mit den Merkmalen der unabhängigen Patentansprüche. Vorteilhafte Weiterbildungen der Erfindung, welche einzeln oder in Kombination realisierbar sind, sind in den abhängigen Ansprüchen dargestellt.

In einem ersten Aspekt der vorliegenden Erfindung wird eine medizinische Vorrichtung zur Durchführung mindestens einer medizinischen Funktion vorgeschlagen. Unter einer medizinischen Vorrichtung ist dabei eine beliebige Vorrichtung zu verstehen, welche eingerichtet ist, um mindestens eine medizinische Funktion durchzuführen, also eine Funktion ausgewählt aus einer therapeutischen, diagnostischen und chirurgischen Funktion. Insbesondere kann es sich bei der medizinischen Funktion um mindestens eine diagnostische Funktion, vorzugsweise eine Messfunktion, und/oder um mindestens eine therapeutische Funktion, insbesondere um mindestens eine Medikationsfunktion, handeln. Insbesondere kann mindestens eine Sensorfunktion vorgesehen sein. Die medizinische Vorrichtung kann insbesondere als persönliche medizinische Vorrichtung ausgestaltet sein und kann ausgestaltet sein, um auf einer Körperoberfläche des Benutzers getragen zu werden. Dementsprechend kann die medizinische Vorrichtung beispielsweise ein Volumen aufweisen, welches vorzugsweise 1000 cm³ nicht überschreitet und vorzugsweise ein Volumen von 50 cm³ nicht überschreitet, insbesondere ein Volumen von 30 cm³ oder sogar 15 cm³ nicht überschreitet.

Die medizinische Vorrichtung umfasst mindestens eine auf einer Körperoberfläche eines Benutzers aufbringbares Steuerteil. Das Steuerteil kann dabei unmittelbar auf einer Haut des Benutzers aufgebracht sein oder auch lediglich indirekt auf die Körperoberfläche aufbringbar sein, beispielsweise unter Zwischenschaltung eines oder mehrerer Elemente wie beispielsweise Gewebeschichten, Stoffschichten, Kunststoffschichten oder ähnlichen Schichten. Besonders bevorzugt ist es jedoch, wenn die medizinische Vorrichtung direkt auf die Körperoberfläche aufbringbar ist.

Unter einem Steuerteil ist dabei allgemein ein Element der medizinischen Vorrichtung zu verstehen, welches einteilig oder auch mehrteilig ausgebildet sein kann, und welches eingerichtet ist, um mindestens eine Ansteuer- und/oder Auswertefunktion der medizinischen Vorrichtung zu erfüllen. Beispielsweise kann es sich dabei um mindestens eine elektronische Funktion handeln, beispielsweise mindestens eine Messfunktion. Zu diesem Zweck kann das Steuerteil beispielsweise mindestens eine Messvorrichtung aufweisen. Alternativ oder zusätzlich kann das Steuerteil auch mindestens eine andere Funktion ausüben, beispielsweise mindestens eine mikromechanische Funktion und/oder Aktorfunktion, beispielsweise die Funktion einer Medikationspumpe. Auch andere Ausgestaltungen sind möglich. Das Steuerteil kann dementsprechend insbesondere mindestens ein elektronisches Bauelement umfassen, insbesondere mindestens ein aktives elektronisches Bauelement, insbesondere mindestens einen hochohmigen Operationsverstärker und/oder mindestens einen Potentiostaten und/oder mindestens einen integrierten Schaltkreis, insbesondere mindestens einen ASIC (applikationsspezifischer integrierter Schaltkreis). Das Steuerteil kann, wie unten noch näher ausgeführt, insbesondere mindestens einen Einwegteil (Disposable) und mindestens einen Mehrwegteil (Reusable) umfassen, wobei der Einwegteil beispielsweise ein unten noch näher beschriebenes Basisteil umfassen kann und wobei der Mehrwegteil beispielsweise mindestens eine Ansteuereinheit umfassen kann, beispielsweise eine Ansteuereinheit, welche eine oder mehrere der oben beschriebenen elektronischen Komponenten umfasst. In dem Einwegteil sind vorzugsweise keine elektronischen Bauelemente, zumindest keine aktiven elektronischen Bauelemente angeordnet, wobei jedoch beispielsweise mindestens eine Batterie und/oder eine andere Art von elektrischem Energiespeicher und/oder mindestens ein Speicherbauelement in dem Einwegteil aufgenommen sein können.

Die medizinische Vorrichtung umfasst weiterhin an mindestens einer Insertionsstelle in ein Körpergewebe des Benutzers insertierbares Funktionselement. Unter einem Funktionselement ist dabei ein Element zu verstehen, welches eingerichtet ist, um die mindestens eine medizinische Funktion durchzuführen und/oder zu unterstützen. Insbesondere kann das Funktionselement mindestens ein Messelement umfassen, beispielsweise mindestens ein Sensorelement zur qualitativen und/oder quantitativen Erfassung mindestens eines Analyten, beispielsweise mindestens eines Metaboliten, in mindestens einer Körperflüssigkeit des Benutzers. Insbesondere kann das insertierbare Funktionselement mindestens einen elektrochemischen Sensor umfassen. Alternativ oder zusätzlich kann das insertierbare Funktionselement jedoch auch mindestens einen Aktor und/oder mindestens einen Teil einer Medikationsvorrichtung umfassen, beispielsweise eine Kanüle. Verschiedene Beispiele werden unten noch näher genannt. Als insertierbar wird ein Element im Rahmen der vorliegenden Erfindung allgemein dann verstanden, wenn dieses Element ganz oder teilweise in ein Körpergewebe des Benutzers einbringbar ist, wobei beispielsweise transkutane oder auch subkutane Einbringungen möglich sind. Insbesondere kann eine Insertierbarkeit eine biokompatible Oberfläche umfassen, beispielsweise eine Oberfläche, welche eingerichtet ist, um über mehrere Stunden, mehrere Tage, mehrere Wochen oder sogar mehrere Monate im Körpergewebe zu verbleiben, ohne Entzündungsreaktionen und/oder allergische Reaktionen hervorzurufen und/oder ohne toxische Einflüsse auf das Körpergewebe auszuüben. Beispielsweise kann das insertierbare Funktionselement zu diesem Zweck mindestens eine inerte Beschichtung umfassen. Auch andere Ausgestaltungen insertierbarer Funktionselemente sind möglich. Das insertierbare Funktionselement kann insbesondere derart ausgestaltet sein, dass sich dieses im insertierten Zustand in einen insertierten Teil und einen durch die Körperoberfläche des Körpers hindurch aus dem Körpergewebe heraus erstreckenden Teil unterteilt. So kann das insertierbare Funktionselement beispielsweise als langgestrecktes Funktionselement ausgestaltet sein, beispielsweise in Form eines needle-type-Sensors und/oder in Form einer Kanüle. Verschiedene andere Ausgestaltungen sind möglich.

Das Funktionselement ist mit dem Steuerteil verbindbar, was die Möglichkeit einer Herstellung einer Verbindung oder auch einen bereits verbundenen Zustand umfassen kann. So kann das Funktionselement insbesondere mit einem Disposable und/oder einem Reusable des Steuerteils verbindbar sein. Der Begriff der Verbindbarkeit umfasst grundsätzlich formschlüssige und/oder stoffschlüssige und/oder kraftschlüssige Verbindungen. Auch Kombinationen der genannten Verbindungen sind möglich. So kann beispielsweise das Funktionselement eingerichtet sein, um über mindestens eine kraftschlüssige und/oder formschlüssige erste Verbindung mit einem Basisteil des Steuerteils verbunden zu werden und anschließend über mindestens eine weitere formschlüssige und/oder kraftschlüssige Verbindung mit einer Ansteuereinheit des Steuerteils verbunden zu werden. Der Begriff der Verbindbarkeit umfasst dabei die Möglichkeit einer reversiblen Verbindbarkeit. Weiterhin umfasst der Begriff "verbindbar" auch eine Ausgestaltung, bei welcher das insertierbare Funktionselement dauerhaft mit dem Steuerteil verbunden ist.

Das Funktionselement ist eingerichtet, um mindestens eine medizinische Funktion durchzuführen. Bezüglich der medizinischen Funktion kann auf die obige Beschreibung möglicher medizinischer Funktionen der medizinischen Vorrichtung verwiesen werden. Die medizinische Funktion kann beispielsweise eine Messfunktion und/oder eine Aktorfunktion sein, beispielsweise eine Sensorfunktion, insbesondere eine elektrochemische Sensorfunktion, und/oder eine Medikationsfunktion, beispielsweise die Funktion einer Verabreichung und/oder Applikation eines Medikaments mittels beispielsweise mindestens einer Kanüle. Auch andere Ausgestaltungen sind möglich.

Das Steuerteil weist mindestens ein Basisteil mit mindestens einer der Körperfläche zuweisenden Auflagefläche auf. Diese Auflagefläche kann im Gebrauchszustand der medizinischen Vorrichtung beispielsweise direkt oder indirekt auf einer Körperoberfläche des Benutzers aufliegen, beispielsweise im Bauchbereich, im Armbereich oder im Rückenbereich des menschlichen oder tierischen Benutzers. Die Auflagefläche kann insbesondere im Wesentlichen als ebene Auflagefläche ausgestaltet sein. Auch gekrümmte oder raue Auflageflächen sind jedoch grundsätzlich möglich. Die Auflagefläche kann beispielsweise im Wesentlichen eben ausgestaltet sein, mit Rauigkeiten, welche vorzugsweise 2-3 mm nicht überschreiten, und/oder kann vorzugsweise eine Fläche aufweisen, welche mindestens 2 cm² beträgt, beispielsweise 2-50 cm², vorzugsweise 3-10 cm². Das Basisteil kann insbesondere als Kunststoffbauteil und/oder als metallisches Bauteil ausgestaltet sein. Beispielsweise kann das Basisteil als im Wesentlichen starres Spritzgussbauteil aus einem thermoplastischen Kunststoff hergestellt sein, wobei unter "im Wesentlichen starr" eine Ausgestaltung verstanden wird, bei der sich das Bauelement zumindest unter Einwirkung seiner eigenen Gewichtskraft und vorzugsweise auch unter Einwirkung von im Gebrauch üblichen Kräften nicht makroskopisch verformt. Auch andere Ausgestaltungen sind jedoch grundsätzlich möglich.

Zur Lösung der oben beschriebenen Problematik wird vorgeschlagen, dass das Basisteil mindestens einen Aufnahmekanal zum Aufnehmen von aus der Insertionsstelle austretender Körperflüssigkeit aufweist. Unter einem Aufnehmen kann dabei im Rahmen der vorliegenden Erfindung jegliche Art der vollständigen oder teilweisen Befüllung des Aufnahmekanals mit der Körperflüssigkeit verstanden werden, welche vorübergehend oder auch dauerhaft erfolgen kann. So kann der Aufnahmekanal als Reservoir zur vorübergehenden oder auch dauerhaften Aufnahme wirken, oder kann auch lediglich zur Ableitung der Körperflüssigkeit dienen, beispielsweise unter Einwirkung von Kapillarkräften und/oder anderen Kräften, wie beispielsweise einer Gewichtskraft der Körperflüssigkeit. Unter einem Aufnahmekanal wird dabei ein Kanal verstanden, welcher allseitig oder zumindest auf drei Seiten von Kanalwänden umgeben ist und welcher eingerichtet ist, um austretende Körperflüssigkeit zu leiten, vorzugsweise von der Insertionsstelle wegzuleiten. Im Gegensatz zu dem oben beschriebenen Stand der Technik soll der Aufnahmekanal eingerichtet sein, um aus der Insertionsstelle austretende Körperflüssigkeit aufzunehmen und vorzugsweise von dieser wegzuleiten, wohingegen aus dem Stand der Technik lediglich Vorrichtungen bekannt sind, bei welchen Körperflüssigkeit aus dem Inneren des Körpergewebes herausgeleitet wird. Dementsprechend kann der Aufnahmekanal beispielsweise mindestens eine Kanalöffnung aufweisen, an welcher die Körperflüssigkeit in den Aufnahmekanal gelangen kann und welche vorzugsweise nicht weiter als 10 mm von der Insertionsstelle entfernt ist, insbesondere nicht mehr als 7mm und besonders bevorzugt nicht mehr als 5 mm oder sogar nicht mehr als 3 mm oder nicht mehr als 2 mm. Der Aufnahmekanal kann insbesondere zumindest teilweise in der Auflagefläche ausgebildet sein. Der Aufnahmekanal kann insbesondere zumindest teilweise parallel zu der Auflagefläche ausgebildet sein oder mit einem Winkel von nicht mehr als 20°, vorzugsweise von nicht mehr als 10° und besonders bevorzugt nicht mehr als 5° zur Auflagefläche. Dementsprechend kann der Aufnahmekanal beispielsweise in einem Zustand, in dem die Auflagefläche direkt oder indirekt auf einer Hautoberfläche des Benutzers aufgebracht ist, im Wesentlichen parallel zu der Hautoberfläche angeordnet sein, wobei beispielsweise die zuvor genannten Winkelabweichungen noch tolerabel sein können. Insbesondere kann der Aufnahmekanal zur Auflagefläche hin geöffnet sein, beispielsweise in Form eines zumindest teilweise geöffneten Kanals. So kann der Aufnahmekanal beispielsweise mindestens eine Vertiefung in der Auflagefläche umfassen, welche beispielsweise mittels einer Spritzgussform oder eines anderen formgebenden Werkzeugs oder mittels einer nachträglichen Einbringung, beispielsweise durch spanabhebende Verfahren und/oder Laserverfahren, in die Auflagefläche eingebracht seien können. Alternativ oder zusätzlich zu einem in einer Richtung quer zur Längserstreckungsrichtung geöffneten Aufnahmekanal ist jedoch grundsätzlich auch ein allseitig quer zur Längserstreckungsrichtung geschlossener Aufnahmekanal möglich. Besonders bevorzugt ist jedoch die Ausgestaltung des Aufnahmekanals als geöffneter Kanal, beispielsweise zumindest teilweise als Spalt und/oder Vertiefung.

Die Auflagefläche kann insbesondere auf der der Körperfläche zuweisenden Seite durch mindestens ein Fixierelement abgedeckt sein, welches der Fixierung des Basisteils auf der Körperoberfläche dient. Dieses Fixierelement kann Bestandteil der medizinischen Vorrichtung sein, insbesondere des Steuerteils und dort vorzugsweise des Einwegteils (Disposable). Das Fixierelement, beispielsweise ein Pflaster, kann die Auflagefläche insbesondere vollständig oder teilweise abdecken und kann optional lateral auch über die Auflagefläche hinausragen. Das Fixierelement kann dementsprechend insbesondere zumindest in einem oder mehreren Bereichen zwischen dem Aufnahmekanal und der Hautoberfläche des Benutzers angeordnet sein. Insbesondere kann das Fixierelement mindestens ein Pflaster umfassen, mit einer der Körperoberfläche zuweisenden Klebeseite. Auch andere Arten der Fixierung des Basisteils auf der Körperoberfläche sind jedoch grundsätzlich möglich, beispielsweise indem das Basisteil selbst eine selbstklebende Auflagefläche aufweist. Deckt das Fixierelement, insbesondere das Pflaster, die Auflagefläche vollständig oder teilweise ab, so kann das Fixierelement den Aufnahmekanal zumindest teilweise abdecken. Auf diese Weise kann insbesondere ein geöffneter Aufnahmekanal durch das Fixierelement vollständig oder teilweise geschlossen werden. Das Fixierelement kann beispielsweise aus einem textilen Material und/oder Kunststoffmaterial und/oder Papiermaterial hergestellt sein. So kann das Fixierelement beispielsweise selbst saugfähige Eigenschaften aufweisen und kann beispielsweise auch ausgestaltet sein, um selbst Körperflüssigkeit, die ihm von dem Aufnahmekanal zugeleitet wird, aufzunehmen. Alternativ oder zusätzlich kann die der Auflagefläche zuweisende Seite des Fixierelements auch inert und nicht aufnahmefähig für die Körperflüssigkeit ausgestaltet sein, sodass beispielsweise diese Seite eine vollständige Kanalwand des Aufnahmekanals bilden kann, welche die Körperflüssigkeit nicht aufnimmt, sodass die Körperflüssigkeit weiter durch den Aufnahmekanal geleitet wird. Allgemein kann die Kanalwand eben ausgestaltet sein, beispielsweise senkrecht zur Auflagefläche. Auch andere Gestaltungen der Kanalwand sind jedoch möglich, beispielsweise schräge Ausgestaltungen und/oder runde Ausgestaltungen und/oder gestufte Ausgestaltungen der Kanalwand, beispielsweise mit einer Stufe.

Das Funktionselement kann, eingerichtet sein, um die mindestens eine medizinische Funktion der medizinischen Vorrichtung durchzuführen und/oder zumindest zu unterstützten. So kann das Funktionselement insbesondere ausgewählt sein aus: einem Sensorelement zur Erfassung mindestens eines Körperzustands des Benutzers; einem Sensorelement zur Erfassung mindestens eines Analyten einer Körperflüssigkeit des Benutzers; einem Aktor zur Beeinflussung mindestens eines Körperzustands des Benutzers; einer Medikationsvorrichtung zur Applikation mindestens eines Wirkstoffs in einem Körpergewebe des Benutzers. Auch Kombinationen der genannten Ausgestaltungen sind möglich, da beispielsweise die Erfassung eines Analyten gleichzeitig auch die Erfassung eines Körperzustands des Benutzers beinhaltet oder da beispielsweise auch eine Medikationsvorrichtung als Aktor zur Beeinflussung eines Körperzustands angesehen oder eingesetzt werden kann. Auch eine Kombination mindestens eines Sensorelements mit mindestens einer Medikationsvorrichtung ist möglich.

Weitere mögliche Ausgestaltungen der Erfindung betreffen die möglichen Ausgestaltungen des Aufnahmekanals. Es können ein oder mehrere Aufnahmekanäle vorgesehen sein. Der Aufnahmekanal kann insbesondere an mindestens einer Kante der Auflagefläche enden. So kann beispielsweise die Auflagefläche einen polygonalen und/oder einen runden, beispielsweise einen ovalen Umfang aufweisen, mit einer umfangsseitigen Kante, an welcher der mindestens eine Aufnahmekanal endet. Auf diese Weise kann der Aufnahmekanal sich beispielsweise von der Insertionsstelle oder von einer Öffnung des Aufnahmekanals in der Nähe der Insertionsstelle bis hin zu einer Kante der Auflagefläche am Umfang der Auflagefläche erstrecken, um die Körperflüssigkeit weit von der Insertionsstelle wegzuleiten.

Die medizinische Vorrichtung kann insbesondere eingerichtet sein, um das insertierbare Funktionselement in einem schräg zur Körperfläche insertierten Zustand zu fixieren. Beispielsweise kann das Funktionselement unter einem Winkel von 45°, wobei auch beispielsweise Abweichungen von vorzugsweise nicht mehr als +/- 20°, insbesondere von nicht mehr als +/- 10° tolerierbar sind, an der Insertionsstelle in das Körpergewebe eintreten. Der Aufnahmekanal kann insbesondere quer zu einer Projektion des insertierten Funktionselements auf die Körperoberfläche angeordnet sein. Beispielsweise kann der Aufnahmekanal unter einem Winkel von den wesentlichen 90° zur Projektion des insertierten Funktionselements auf die Körperoberfläche angeordnet sein, vorzugsweise unter einem im Wesentlichen rechten Winkel, also beispielsweise unter einem Winkel zwischen 70° und 110°, insbesondere zwischen 80° und 100° und besonders bevorzugt in einem Winkel von 90°.

Das Basisteil kann in der Auflagefläche insbesondere mindestens eine Öffnung aufweisen, welche vollständig oder teilweise von dem Basisteil umschlossen sein kann. Die Öffnung kann beispielsweise eine polygonale, eine runde und insbesondere eine ovale Gestalt aufweisen. Das insertierbare Funktionselement durchdringt im insertierten Zustand diese Öffnung. Der Aufnahmekanal erstreckt sich dann vorzugsweise von der Öffnung, also von einem der Insertionsstelle zuweisenden inneren Rand der Öffnung, aus. Beispielsweise kann sich der Aufnahmekanal von der Öffnung aus zu einer äußeren Kante der Auflagefläche erstrecken. Der Aufnahmekanal kann einteilig oder mehrteilig ausgestaltet sein. Insbesondere kann sich der Aufnahmekanal von der Öffnung aus in mindestens zwei Richtungen erstrecken. Beispielsweise kann sich der Aufnahmekanal in zwei entgegengesetzten Richtungen erstrecken, welche im Wesentlichen senkrecht zur Projektion des Funktionselements im insertierten Zustand auf die Körperoberfläche erstrecken, gemäß der obigen Definition. Durch diese Erstreckung in mindestens zwei Richtungen kann auch eine Umkehrbarkeit einer Aufbringung auf eine Körperoberfläche gewährleistet werden, beispielsweise indem sowohl eine rechtshändige Aufbringung in einer ersten Orientierung als auch eine linkshändige Aufbringung in einer zweiten Orientierung erfolgen kann.

Besonders bevorzugt ist es, wenn mindestens eine Seitenwand des Aufnahmekanals, welche eine Ausbreitung der Körperflüssigkeit in einer Richtung parallel zur Auflagefläche begrenzt, in der Öffnung mündet und/oder die Öffnung zumindest teilweise umschließt. Die Öffnung kann auch vollständig innerhalb des Aufnahmekanals angeordnet sein. Der Aufnahmekanal kann in einem Bereich außerhalb der Öffnung, beispielsweise in einem Abstand von mindestens 3 mm von der Öffnung, insbesondere im Wesentlichen parallele Seitenwände aufweisen. Der Aufnahmekanal kann weiterhin insbesondere auch mindestens eine Erweiterung als Reservoir für die Körperflüssigkeit aufweisen. Beispielsweise können ein oder mehrere Erweiterungen vorgesehen sein. Alternativ kann der Aufnahmekanal jedoch außerhalb der Öffnung auch ausschließlich als Aufnahmekanal mit parallelen Seitenwänden ausgestaltet sein. Der Aufnahmekanal kann insbesondere als Kapillarkanal ausgestaltet sein, d.h. als Kanal, welcher bei typischen Körperflüssigkeiten wie Blut oder interstitieller Flüssigkeit Kapillarkräfte entwickelt. Der Aufnahmekanal kann insbesondere eine Tiefe senkrecht zu der Auflagefläche aufweisen, wobei die Tiefe 0,05 mm bis 3 mm betragen kann, insbesondere 0,1 mm bis 2 mm und besonders bevorzugt 0,2 mm bis 1 mm, beispielsweise 0,5 mm. Der Aufnahmekanal kann an seiner engsten Stelle, beispielsweise an einer Stelle, an welcher die Seitenwände im Wesentlichen parallel verlaufen, insbesondere eine Breite von 0,2 mm bis 10 mm aufweisen, insbesondere von 0,3 mm bis 5 mm und besonders bevorzugt eine Breite von 0,5 mm bis 2 mm, beispielsweise 1 mm.

Zur Ausbildung des Aufnahmekanals in dem Basisteil, insbesondere in der Auflagefläche, sind verschiedene Möglichkeiten gegeben und im Rahmen der vorliegenden Erfindung realisierbar. So kann das Basisteil beispielsweise mindestens einen Vorsprung aufweisen, beispielsweise einen langgestreckten Vorsprung, welcher sich von der Auflagefläche aus erstreckt und welcher zumindest einen Teil mindestens einer Seitenwand des Aufnahmekanals bilden kann. Beispielsweise kann dieser Vorsprung eine Höhe von 0,2 mm bis 2 mm, insbesondere von 0,5 mm bis 1 mm, aufweisen. Ein derartiger Vorsprung, welcher beispielsweise als Wulst, Wall oder Rippe ausgebildet sein kann und welcher insbesondere als langgestreckte Seitenwand oder ein Teil derselben fungieren kann, ist kunststofftechnisch leicht herstellbar. Der Vorsprung kann auch beispielsweise, alternativ oder zusätzlich, weiteren Zwecken dienen. So kann der mindestens eine Vorsprung beispielsweise auch eine Abdichtwirkung aufweisen und beispielsweise als Abdichtvorsprung oder als Abdichtrippe ausgestaltet sein. Alternativ oder zusätzlich kann der Vorsprung auch als Verbindungselement der Auflagefläche mit einem oder mehreren weiteren Elementen dienen, beispielsweise zur Verbindung der Auflagefläche mit dem Fixierelement, beispielsweise einem Pflaster und/oder Vlies. So kann der mindestens eine Vorsprung beispielsweise mit dem Fixierelement verklebt, verschweißt oder auf andere Weise verbunden sein.

Alternativ oder zusätzlich zu mindestens einem Vorsprung als Seitenwand des Aufnahmekanals kann der Aufnahmekanal auch zumindest teilweise durch mindestens eine sich in die Auflagefläche hinein erstreckende Vertiefung gebildet werden. Insbesondere kann diese Vertiefung mindestens einen Kapillarkanal bilden. Diese Vertiefung kann beispielsweise eine langgestreckte Nut, Rille oder ähnliche Vertiefung sein, mit einem beispielsweise rechteckigen Querschnitt, einem polygonalen Querschnitt, einem runden Querschnitt oder auch einem dreieckigen oder auf andere Weise gestalteten Querschnitt in einer Schnittebene senkrecht zur Auflagefläche.

Wie oben dargestellt, kann das Steuerteil insbesondere mehrteilig ausgestaltet sein und kann mindestens ein Disposable und mindestens ein Reusable aufweisen. So kann das Basisteil und optional auch das mindestens eine Fixierelement, beispielsweise das mindestens eine Pflaster, Teil des Disposables sein. Zusätzlich kann das Steuerteil auch mindestens ein Reusable aufweisen, beispielsweise mindestens eine Ansteuereinheit. Diese Ansteuereinheit kann insbesondere mit dem Basisteil verbindbar sein, beispielsweise mittels mindestens einer formschlüssigen und/oder mindestens einer kraftschlüssigen Verbindung. Zu diesem Zweck können das Basisteil und/oder das Ansteuerteil mindestens ein Verbindungselement aufweisen, um eine Verbindung der genannten Art herzustellen. Insbesondere kann sich das Basisteil mit dem Ansteuerteil zu einem gemeinsamen Steuerteil mit einem gemeinsamen Gehäuse verbinden, welches beispielsweise im Wesentlichen mediendicht, beispielsweise feuchtigkeitsdicht, ausgestaltet sein kann. Das Basisteil und das Ansteuerteil können beispielsweise mittels mindestens einer Steckverbindung miteinander verbunden sein, welche auch eine elektrische Steckverbindung umfassen kann. Auf diese Weise kann beispielsweise in dem Basisteil mindestens ein elektrischer Energiespeicher, beispielsweise mindestens eine Batterie, angeordnet sein, welche mindestens ein elektronisches Bauelement in der Ansteuereinheit mit elektrischer Energie versorgen kann.

Die Ansteuereinheit und das Basisteil sind vorzugsweise reversibel miteinander verbindbar, im Sinne dessen, dass das Basisteil vorzugsweise Teil eines Disposables ist, also eines Einwegteils, welches beispielsweise nach einer Lebensdauer des Funktionselements ausgetauscht werden kann. In einem weiteren Aspekt der vorliegenden Erfindung wird dementsprechend ein Basisteil vorgeschlagen, welches insbesondere als Einwegteil ausgestaltet sein kann und als Verbrauchsmaterial in einer medizinischen Vorrichtung gemäß einer oder mehreren der oben beschriebenen Ausgestaltungen verwendet werden kann. Das Basisteil ist für den Einsatz in einer medizinischen Vorrichtung vorgesehen, wobei insbesondere eine medizinische Vorrichtung gemäß der obigen Beschreibung verwendet werden kann, sodass bezüglich möglicher Ausgestaltungen dieser medizinischen Vorrichtung auf die obige Beschreibung verwiesen werden kann. Auch andere Ausgestaltungen sind jedoch grundsätzlich möglich. Das Basisteil ist eingerichtet, um als Teil mindestens eines Steuerteils der medizinischen Vorrichtung auf einer Körperoberfläche eines Benutzers aufgebracht zu werden. Mindestens ein an mindestens einer Insertionsstelle in ein Körpergewebe des Benutzers insertierbares Funktionselement ist mit dem Steuerteil, insbesondere dem Basisteil, verbindbar. Zu diesem Zweck kann das Basisteil beispielsweise mindestens ein Verbindungselement umfassen, beispielsweise ein Verbindungselement zur kraftschlüssigen und/oder formschlüssigen Verbindung mit dem insertierbaren Funktionselement. Alternativ oder zusätzlich sind jedoch auch andere Verbindungsarten möglich. Das Funktionselement ist eingerichtet, um mindestens eine medizinische Funktion durchzuführen. Das Funktionselement kann auch einstückig mit dem Basisteil ausgebildet sein, also permanent mit dem Basisteil verbunden sein. Das Basisteil weist mindestens eine der Körperoberfläche zuweisende Auflagefläche auf. Das Basisteil weist weiterhin mindestens einen Aufnahmekanal zum Aufnehmen von aus der Insertionsstelle austretender Körperflüssigkeit auf. Für weitere mögliche Ausgestaltungen kann auf die obige Beschreibung verwiesen werden.

Die oben beschriebene medizinische Vorrichtung und das oben beschriebene Basisteil weisen gegenüber bekannten medizinischen Vorrichtungen und Bestandteilen desselben zahlreiche Vorteile auf. So wird, im Gegensatz zum oben zitierten Stand der Technik, insbesondere aus der Insertionsstelle austretende Körperflüssigkeit in dem Aufnahmekanal aufgenommen. Ein Austritt zusätzlicher Körperflüssigkeit aus dem Körpergewebe wird jedoch nicht forciert. Weiterhin kann die medizinische Vorrichtung, insbesondere das Basisteil, sehr klein ausgestaltet sein, da für den Aufnahmekanal grundsätzlich kein zusätzlicher Bauraum benötigt wird. Der Aufnahmekanal, welcher in dem Basisteil ausgebildet ist, kann als separater Kanal, unabhängig von dem Sensor und/oder der Kanüle beziehungsweise dem Funktionselement ausgestaltet sein und beispielsweise getrennt gehandhabt und/oder hergestellt werden. Der Aufnahmekanal kann insbesondere in einem Bodymount ausgebildet sein, da das Basisteil Bestandteil eines auf der Körperoberfläche aufbringbaren Elements der medizinischen Vorrichtung sein kann. Der Aufnahmekanal ist geeignet, um beispielsweise überschüssiges Blut bzw. Flüssigkeit von der Insertionsstelle abzuleiten. Der Aufnahmekanal kann sich beispielsweise sowohl rechts als auch links von einer Öffnung in der Auflagefläche aus erstrecken, in welcher das Funktionselement, beispielsweise der Sensor, nach der Insertion aufgenommen ist. Auf diese Weise kann beispielsweise Blut oder interstitielle Flüssigkeit, welche beim Insertionsvorgang an der Insertionsstelle austreten können, von dieser Austrittsstelle und von den elektrischen Kontakten in der Nachbarschaft weggeleitet werden, insbesondere um Leckströme zu vermeiden. Auf diese Weise wird mittels der vorgeschlagenen Erfindung auch die Funktionssicherheit der medizinischen Vorrichtung erhöht, da insbesondere Leckströme, Kriechströme oder andere elektrische Unsicherheiten und Fehler zuverlässig vermieden werden können. Gleichzeitig kann durch die separate Ausgestaltung des Aufnahmekanals, vorzugsweise separat von dem Funktionselement und vorzugsweise trennbar von dem Funktionselement, für eine Austauschbarkeit des Aufnahmekanals gesorgt werden, beispielsweise im Rahmen eines Austauschs des Disposables. Dementsprechend kann für eine gezielte und hygienische Entsorgung der Körperflüssigkeit gesorgt werden. Insbesondere kann zwischen der Auflagefläche des Basisteils, welches insbesondere als Bodenplatte ausgestaltet sein kann, und dem mindestens einen Fixierelement, beispielsweise dem Pflaster, der mindestens eine Aufnahmekanal als Kapillarspalt ausgestaltet sein, welcher dafür sorgen kann, dass Blut und/oder andere Körperflüssigkeiten gezielt von kritischen Komponenten der medizinischen Vorrichtung weggeführt werden, beispielsweise von einem Dichtungssystem, welches eine Auswerteeinheit und das Basisteil gegeneinander abdichtet, und/oder von elektrischen Kontakten des Funktionselements, beispielsweise des Sensors.

Somit kann nach einer Insertion des Funktionselements insbesondere verhindert werden, dass eine Blutung an der Insertionsstelle nicht die Dichtungen und/oder die elektrischen Kontakte der medizinischen Vorrichtung verschmutzt. Auf diese Weise kann ein häufiges Auswechseln des Bodymounts verhindert werden, was einen Kostenvorteil bedingt. Weiterhin kann auch verhindert werden, dass unbemerkte Blutungen oder durch den Benutzer unbemerktes Austreten anderer Körperflüssigkeiten nicht dazu führen, dass Undichtigkeiten in der medizinischen Vorrichtung, insbesondere in dem Steuerteil, und/oder Kurzschlüsse bzw. Kriechströme in der elektrischen Kontaktierung auftreten. Insbesondere kann weiterhin durch den Aufnahmekanal auch eine Verschmutzung eines Reusables, beispielsweise einer Auswerteeinheit, durch die Körperflüssigkeiten zuverlässig verhindert werden. Weiterhin können auch andere Verunreinigungen zuverlässig verhindert oder zumindest vermindert werden, beispielsweise Verkrustungen durch eingetrocknete Körperflüssigkeit, die ansonsten auch hygienische Probleme verursachen könnten. Die erfindungsgemäße Ausgestaltung kann somit erheblich zu einer Verbesserung der hygienischen Bedingungen der Medizinischen Vorrichtung beitragen.

### Kurze Beschreibung der Figuren

Weitere Merkmale der vorliegenden Erfindung, welche einzeln und auch in beliebiger Kombination realisierbar sind, ergeben sich aus der nachfolgenden Beschreibung von bevorzugten Ausführungsbeispielen, welche in den Figuren dargestellt sind und welche einzeln oder in beliebiger Kombination realisierbar sind. Die Erfindung ist jedoch nicht auf die Ausführungsbeispiele beschränkt. Gleiche Bezugsziffern in den einzelnen Figuren bezeichnen dabei gleiche oder funktionsgleiche bzw. hinsichtlich ihrer Funktion in einander entsprechende Elemente.

Im Einzelnen zeigen:
- Figur 1: Eine Seitenansicht eines bevorzugten Ausführungsbeispiels einer erfindungsgemäßen medizinischen Vorrichtung;
- Figur 2: Ein Basisteil der medizinischen Vorrichtung gemäß Figur 1 in perspektivischer Darstellung;
- Figur 3: Eine Sensoreinheit zum Einsetzen in einer medizinischen Vorrichtung gemäß Figur 1;
- Figur 4: Eine Darstellung eines Basisteils der medizinischen Vorrichtung gemäß Figur 1 in Draufsicht auf eine Auflagefläche; und
- Figur 5: Eine Seitenansicht des Basisteils der Vorrichtung gemäß Figur 1.

### Ausführungsbeispiele

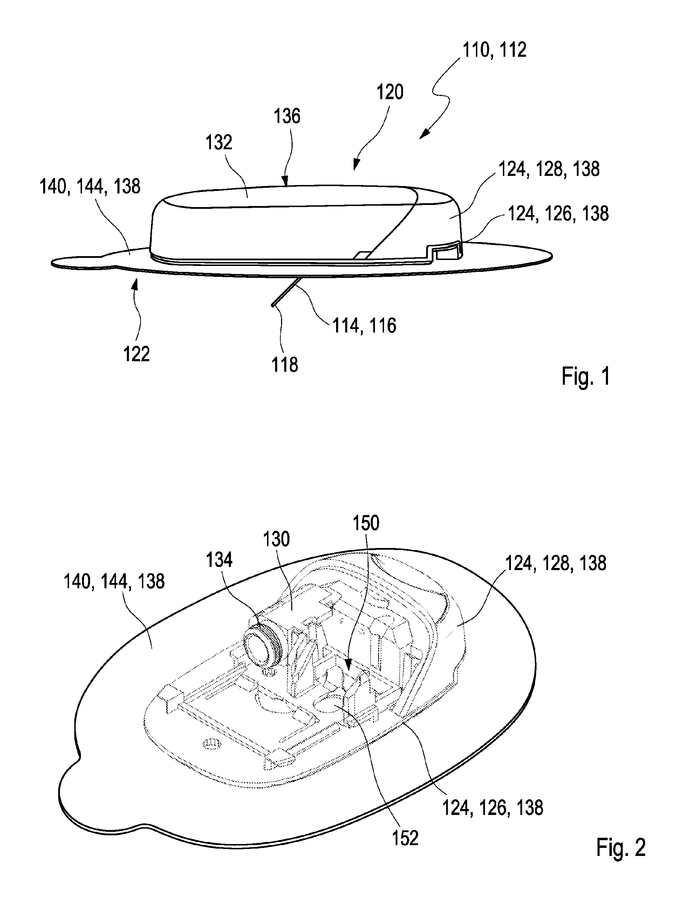

In den Figuren 1-5 sind verschiedene Komponenten einer medizinischen Vorrichtung 110 zur Durchführung mindestens einer medizinischen Funktion dargestellt, auf welche im Folgenden gemeinsam Bezug genommen wird.

In Figur 1 ist diese medizinische Vorrichtung 110 in einer perspektivischen Darstellung von der Seite gezeigt. Die medizinische Vorrichtung 110 ist in diesem Ausführungsbeispiel als Sensorvorrichtung 112 ausgestaltet und für Langzeit-Messungen einer Analytkonzentration in einem Körpergewebe eines Benutzers eingerichtet, beispielsweise für Langzeit-Blutglukosemessungen. Die Sensorvorrichtung 112 umfasst zu diesem Zweck mindestens ein insertierbares Funktionselement 114 in Form eines insertierbaren Sensorelements 116, der mit einem insertierbaren Ende 118 in das Körpergewebe insertiert werden kann. An dem insertierbaren Ende 118 sind beispielsweise zwei oder mehrere Elektroden angeordnet, um die Analytkonzentration elektrochemisch bestimmen zu können.

Weiterhin weist die medizinische Vorrichtung 110 in dem dargestellten Ausführungsbeispiel ein Steuerteil 120 auf, welches in Figur 1 dargestellt ist und welches im insertierten Zustand des Sensorelements 116 außerhalb des Körpergewebes auf einer Körperoberfläche des Benutzers angeordnet ist, mit einer dem der Körperoberfläche zuweisenden Seite 122. Das Steuerteil 120 umfasst ein Basisteil 124, welches häufig auch als Basisplatte 126 bezeichnet wird oder eine Basisplatte 126 umfassen kann. Das Basisteil 124 kann beispielsweise ein Batteriegehäuse 128 umfassen, zur Aufnahme eines oder mehrerer elektrischer Energiespeicher. Weiterhin kann das Basisteil 124 auch, wie in Figur 2 gezeigt, mindestens ein Verbindungselement 130 umfassen, mittels dessen das Basisteil 124 mit mindestens einer Ansteuereinheit 132 des Steuerteils 120 verbindbar ist, beispielsweise über eine Steckverbindung. Das Verbindungselement 130, wobei auch mehrere Verbindungselemente vorgesehen sein können, kann insbesondere mindestens ein Dichtelement 134 umfassen, beispielsweise mindestens einen O-Ring, um eine feuchtigkeitsdichte Ausgestaltung der Verbindung zwischen dem Basisteil 124 und der Ansteuereinheit 132 zu gewährleisten.

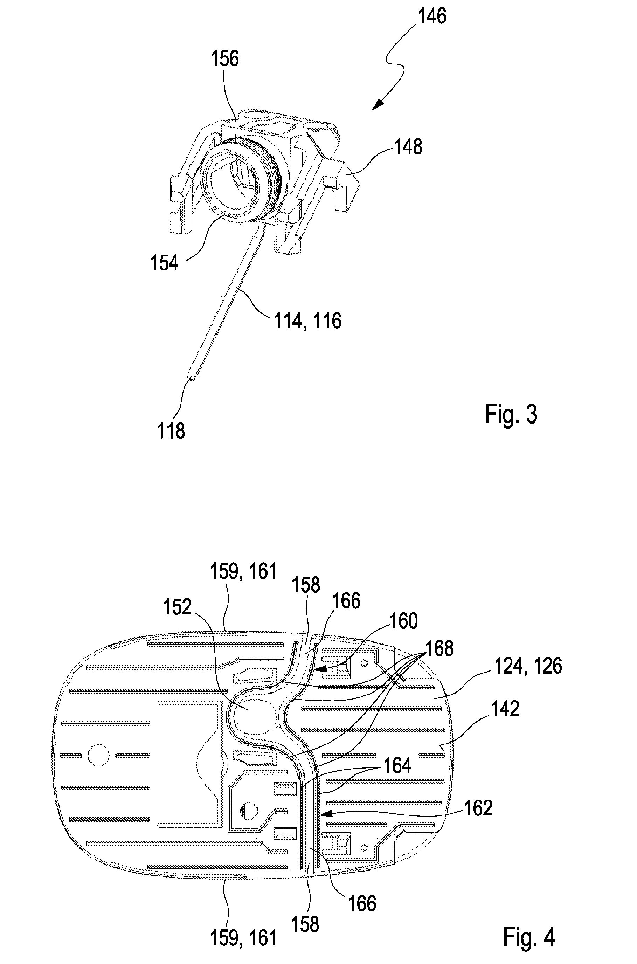

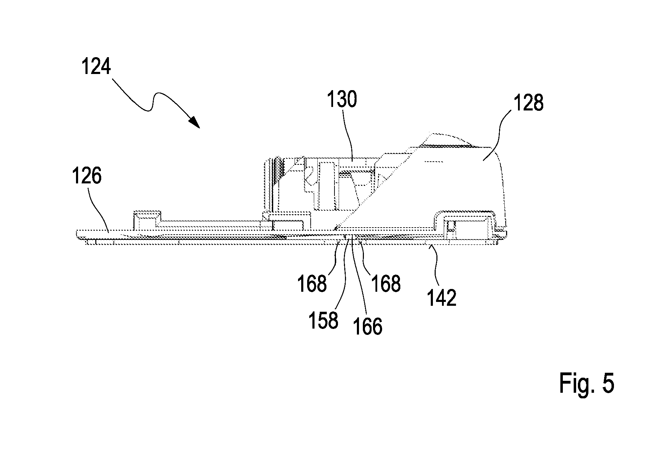

Die Ansteuereinheit 132 kann beispielsweise ein oder mehrere elektrische Ansteuer-und/oder Auswertekomponenten umfassen, um Signale des Sensorelements 116 zu erfassen und/oder zu speichern und/oder zumindest teilweise auszuwerten. So kann beispielsweise in der Ansteuereinheit 132 mindestens ein Potentiostat vorgesehen sein, um elektrochemische Messungen mittels des Sensorelements 116 durchzuführen. Die Ansteuereinheit 132 kann dementsprechend beispielsweise als Reusable 136, also als Mehrwegteil, ausgestaltet sein und für mindestens zwei, vorzugsweise mindestens 10 Messvorgänge, welche jeweils über mehrere Tage, mehrere Wochen oder sogar mehrere Monate dauern können, verwendet werden. Das Basisteil 124 ist hingegen Bestandteil eines Disposables (Einwegteil) 138, welches in Figur 2 dargestellt ist und welches insbesondere auch als sogenanntes Bodymount bezeichnet werden kann. Dieses kann neben dem Basisteil 124 weiterhin mindestens ein Fixierelement 140 umfassen, welches zwischen einer der Körperoberfläche des Benutzers zuweisenden Auflagefläche 142 (siehe die Draufsicht in Figur 4 und die Seitenansicht des Basisteils 124 in Figur 5) angeordnet ist. Dieses Fixierelement 140 kann beispielsweise ganz oder teilweise als Pflaster 144 ausgestaltet sein.

Mit dem Steuerteil 120, insbesondere im vorliegenden Beispiel mit dem Basisteil 124, ist eine Sensoreinheit 146 verbindbar. Diese Sensoreinheit 146 ist in Figur 3 in einer perspektivischen Darstellung gezeigt. Sie umfasst neben dem Sensorelement 116 einen Sensorhalter 148, welcher während der Insertion mit einer entsprechenden Halterung 150 in dem Basisteil 124 verbindbar ist, beispielsweise formschlüssig und/oder kraftschlüssig. Bei dieser Insertion, welche beispielsweise mittels mindestens eines Insertionsbestecks und/oder mindestens einer Insertionskanüle bzw. Insertionsnadel erfolgt, wird das Sensorelement 116 durch eine Öffnung 152 in dem Basisteil 124 geschoben und in das Körpergewebe insertiert, sodass eine Insertionsstelle (in den Figuren nicht dargestellt) innerhalb der Öffnung 152 angeordnet ist. Der Sensorhalter 148 wird dabei mit der Halterung 150 verbunden. Wie in Figur 3 erkennbar, weist auch der Sensorhalter 148 ein Verbindungselement 154 auf, welches wiederum über ein Dichtelement 156 verfügt. Bei mit dem Basisteil 124 verbundenem Sensorhalter 148, also nach dem Insertionsvorgang, umfasst das bis dahin fertiggestellte Bauelement somit neben dem Basisteil 124 und dem Fixierelement 140 die Sensoreinheit 146 als gemeinsame Baugruppe, welche anschließend mit der Ansteuereinheit 132 verbunden wird.

Wie oben beschrieben, umfasst das Basisteil 124 erfindungsgemäß mindestens einen Aufnahmekanal zum Aufnehmen von Körperflüssigkeiten. Dieser Aufnahmekanal ist in einer Aufsicht auf die Auflagefläche 142 gemäß Figur 4 und in einer Seitenansicht des Basisteils 124 in Figur 5 erkennbar und dort mit der Bezugsziffer 158 bezeichnet. Der Aufnahmekanal 158 erstreckt sich auf der Auflagefläche 142 in dem dargestellten Ausführungsbeispiel beidseitig der Öffnung 152 und weist zwei Arme 160, 162 auf. Der Aufnahmekanal 158 kann sich beispielsweise von der Öffnung 152 aus hin zu einer äußeren Kante 159 des Basisteils 124 an einem Rand 161 der Auflagefläche 142 erstrecken. Der Aufnahmekanal 158 ist außerhalb der Öffnung 152 als Kanal mit im Wesentlichen parallelen Seitenwänden 164 ausgestaltet. Diese Seitenwände 164 werden einerseits dadurch gebildet, dass der Aufnahmekanal 158 eine Vertiefung 166 in Form einer Nut umfasst, welche sich in die Auflagefläche 162 hinein erstreckt. Alternativ oder zusätzlich kann der Aufnahmekanal 158 jedoch auch ganz oder teilweise dadurch gebildet werden, dass langgestreckte Vorsprünge 168 vorgesehen sind, welche ebenfalls Seitenwände 164 des Aufnahmekanals 158 bilden können und welche beispielsweise, wie in Figur 4 erkennbar, die Öffnung 152 vollständig oder teilweise umgeben können. Die Seitenwände 164 können also gestuft ausgestaltet sein. Die oben angegebenen bevorzugten Breiten des Aufnahmekanals 158 können sich dann beispielsweise auf eine mittlere Breite beziehen. Auch andere Ausgestaltungen des Aufnahmekanals 158 sind jedoch grundsätzlich möglich.

Eine medizinische Vorrichtung 118 gemäß dem in den Figuren 1-5 dargestellten Ausführungsbeispiel wurde in einem Praxisversuch getestet. Dabei wurden Dummy-Sensorelemente 116 mit einer praxistauglichen Geometrie verwendet, sowie eine Kunsthaut und Kunstblut mit 150 ml/dl Glukose. Zur Beobachtung der Vorgänge im Bereich der Insertionsstelle wurden ein Digitalmikroskop und eine Videokamera verwendet. Bei den Versuchen wurde untersucht, ob überschüssiges Blut nach der Insertion über den Aufnahmekanal 158, der analog zu einer Kapillaren beispielsweise bei einem Spritzgussvorgang in dem Basisteil 124 hergestellt werden kann, abgeleitet wird. Das Basisteil 124 wurde dabei als Rapid-Prototyping-Teil ausgeführt und mit einem Aufnahmekanal 158 in Form eines Kapillarspalts versehen. Auf die Auflagefläche 142 wurde das Pflaster 144 aufgeklebt. Der Kapillarspalt des Aufnahmekanals 158 wird dabei am Randbereich verklebt, jedoch nicht zugeklebt.

Das derart hergestellte Bodymount wurde auf die Kunsthaut aufgeklebt und diese Versuchsanordnung senkrecht gestellt, wie dies beispielsweise bei einem senkrecht stehenden Benutzer, bei welchem im Bauchbereich ein Sensorelement 116 insertiert ist, der Fall ist. Anschließend wurde eine Spritze mit einer aufgesetzten Kanüle mit Kunstblut gefüllt. Die Spritze wurde von der dem Steuerteil 120 gegenüber liegenden Seite der Kunsthaut durch die Kunsthaut gesteckt, sodass das Kunstblut in die Insertionsbohrung des Bodymounts gefüllt werden konnte. Anschließend wurde ein Sensor insertiert. Daraufhin wurde mit langsamer Geschwindigkeit, welche auf ungefähr 5 µl/s geschätzt wurde, manuell Kunstblut in die Insertionsstelle gespritzt. Das Blut floss entlang des abgeklebten Aufnahmekanals 158 nach unten und auf der Unterseite der Basisplatte 126 auf das Pflaster 144.

Nach dieser Dosierung wurde der Sensorstecker, also die Verbindung durch das Verbindungselement 154 in Figur 3, auf Blutverschmutzungen untersucht. Insbesondere am Dichtelement 156, also beispielsweise am O-Ring, und im Bereich der elektrischen Kontaktierung des Sensorelements 116, welche besonders kritische Stellen darstellen, konnte dabei Blut entdeckt werden. Blutreste waren lediglich am Sensorelement 116 selbst erkennbar. Diese Versuche zeigten, dass der Ablauf von überschüssigem Blut durch den Aufnahmekanal 158 prinzipiell funktioniert. Dies ist ein Vorteil für den Benutzer, der im Falle auch einer starken Blutung nicht das gesamte Bodymount gemäß Figur 2 auswechseln muss. Dem Benutzer entsteht hierdurch ein Kostenvorteil, und das Sensorelement 116 muss nicht erneut insertiert werden.

Ob der Aufnahmekanal 158 tatsächlich Kapillarkräfte entfaltet oder ob lediglich ein Abfließen des Bluts bzw. anderer Körperflüssigkeiten erfolgt, beispielsweise aufgrund einer Schwerkraft, kann dabei dahingestellt bleiben. Insbesondere bei liegenden Patienten bieten sich Aufnahmekanäle 158 an, welche auch eine Kapillarwirkung entfalten, da hier die Schwerkraft eine geringere Rolle spielt. In der Hauptgebrauchslage jedoch, in welcher die Auflagefläche 142 senkrecht steht, können zwar grundsätzlich auch Kapillareffekte auftreten, der Haupteffekt könnte hierbei jedoch durch die Schwerkraft erfolgen, Insbesondere ist es somit von Vorteil, wenn bei der dargestellten medizinischen Vorrichtung 110 sowie anderen Ausführungsbeispielen der medizinischen Vorrichtung 110 der Aufnahmekanal 158 oder mindestens einer der Aufnahmekanäle 158 (wenn mehrere vorgesehen sind) mindestens eine Richtungskomponente hin zur Erdoberfläche aufweist, sodass durch die Schwerkraft getrieben Körperflüssigkeit durch diesen Aufnahmekanal 158 abfließen kann.

### Bezugszeichenliste

- 110: medizinische Vorrichtung
- 112: Sensorvorrichtung
- 114: Funktionselement
- 116: Sensorelement
- 118: insertierbares Ende
- 120: Steuerteil
- 122: der Körperoberfläche zuweisende Seite
- 124: Basisteil
- 126: Basisplatte
- 128: Batteriegehäuse
- 130: Verbindungselement
- 132: Ansteuereinheit
- 134: Dichtelement
- 136: Reusable (Mehrwegteil)
- 138: Disposable (Einwegteil)
- 140: Fixierelement
- 142: Auflagefläche
- 144: Pflaster
- 146: Sensoreinheit
- 148: Sensorhalter
- 150: Halterung
- 152: Öffnung
- 154: Verbindungselement
- 156: Dichtelement
- 158: Aufnahmekanal
- 159: Kante
- 160: Arm
- 161: Rand
- 162: Arm
- 164: Seitenwände
- 166: Vertiefung
- 168: Vorsprung

## Patentansprüche

1. Medizinische Vorrichtung (110) zur Durchführung mindestens einer medizinischen Funktion, umfassend mindestens ein auf einer Körperoberfläche eines Benutzers aufbringbares Steuerteil (120) und mindestens ein an mindestens einer Insertionsstelle in ein Körpergewebe des Benutzers insertierbares Funktionselement (114), wobei das Funktionselement (114) mit dem Steuerteil (120) verbindbar ist, wobei das Funktionselement (114) eingerichtet ist, um mindestens eine medizinische Funktion durchzuführen, wobei das Steuerteil (120) mindestens ein Basisteil (124) mit mindestens einer der Körperoberfläche zuweisenden Auflagefläche (142) aufweist, wobei das Basisteil (124) mindestens einen Aufnahmekanal (158) zum Aufnehmen von aus der Insertionsstelle austretender Körperflüssigkeit aufweist.

2. Medizinische Vorrichtung (110) nach dem vorhergehenden Anspruch, wobei der Aufnahmekanal (158) zumindest teilweise in der Auflagefläche (142) ausgebildet ist.

3. Medizinische Vorrichtung (110) nach dem vorhergehenden Anspruch, wobei die Auflagefläche (142) auf der der Körperoberfläche zuweisenden Seite durch mindestens ein Fixierelement (140) zur Fixierung des Basisteils (124) auf der Körperoberfläche abgedeckt ist, insbesondere mindestens ein Pflaster (144).

4. Medizinische Vorrichtung (110) nach dem vorhergehenden Anspruch, wobei das Fixierelement (140) den Aufnahmekanal (158) zumindest teilweise abdeckt.

5. Medizinische Vorrichtung (110) nach einem der vorhergehenden Ansprüche, wobei das Funktionselement (114) ausgewählt ist aus: einem Sensorelement (116) zur Erfassung mindestens eines Körperzustands des Benutzers; einem Sensorelement (116) zur Erfassung mindestens eines Analyten einer Körperflüssigkeit des Benutzers; einem Aktor zur Beeinflussung mindestens eines Körperzustands des Benutzers; einer Medikationsvorrichtung zur Applikation mindestens eines Wirkstoffs in ein Körpergewebe des Benutzers.

6. Medizinische Vorrichtung (110) nach einem der vorhergehenden Ansprüche, wobei der Aufnahmekanal (158) an mindestens einer Kante (159) der Auflagefläche (142) endet.

7. Medizinische Vorrichtung (110) nach einem der vorhergehenden Ansprüche, wobei das Basisteil (124) in der Auflagefläche (142) mindestens eine Öffnung (152) aufweist, wobei das insertierbare Funktionselement (114) im insertierten Zustand die Öffnung (152) durchdringt, wobei der Aufnahmekanal (158) sich von der Öffnung (152) aus erstreckt.

8. Medizinische Vorrichtung (110) nach dem vorhergehenden Anspruch, wobei sich der Aufnahmekanal (158) von der Öffnung (152) aus in mindestens zwei Richtungen erstreckt.

9. Medizinische Vorrichtung (110) nach einem der beiden vorhergehenden Ansprüche, wobei sich der Aufnahmekanal (158) von der Öffnung (152) aus bis hin zu mindestens einem Rand (161) der Auflagefläche (142) erstreckt.

10. Medizinische Vorrichtung (110) nach einem der vorhergehenden Ansprüche, wobei der Aufnahmekanal (158) als Kapillarkanal ausgebildet ist.

11. Medizinische Vorrichtung (110) nach einem der vorhergehenden Ansprüche, wobei der Aufnahmekanal (158) eine Tiefe senkrecht zu der Auflagefläche (142) aufweist, wobei die Tiefe 0,05 mm bis 3 mm, insbesondere 0,1 mm bis 2 mm und besonders bevorzugt 0,2 mm bis 1 mm beträgt.

12. Medizinische Vorrichtung (110) nach einem der vorhergehenden Ansprüche, wobei der Aufnahmekanal (158) an seiner engsten Stelle eine Breite von 0,2 mm bis 10 mm aufweist, insbesondere von 0,3 mm bis 5 mm und besonders bevorzugt eine Breite von 0,5 mm bis 2 mm.

13. Medizinische Vorrichtung (110) nach einem der vorhergehenden Ansprüche, wobei das Basisteil (124) mindestens einen Vorsprung (168) aufweist, wobei sich der Vorsprung (168) von der Auflagefläche (142) aus erstreckt, wobei der Vorsprung (168) zumindest einen Teil mindestens einer Seitenwand (164) des Aufnahmekanals (158) bildet.

14. Medizinische Vorrichtung (110) nach einem der vorhergehenden Ansprüche, wobei der Aufnahmekanal (158) zumindest teilweise durch mindestens eine sich in die Auflagefläche (142) hinein erstreckende Vertiefung (166) gebildet wird.

15. Basisteil (124), insbesondere als Einwegteil ausgestaltetes Basisteil (124), für den Einsatz in einer medizinischen Vorrichtung (110), insbesondere nach einem der vorhergehenden Ansprüche, wobei das Basisteil (124) eingerichtet ist, um als Teil mindestens eines Steuerteils (120) der medizinischen Vorrichtung (110) auf einer Körperoberfläche eines Benutzers aufgebracht zu werden, wobei mindestens ein an mindestens einer Insertionsstelle in ein Körpergewebe des Benutzers insertierbares Funktionselement (114) mit dem Steuerteil (120), insbesondere dem Basisteil (124), verbindbar ist, wobei das Funktionselement (114) eingerichtet ist, um mindestens eine medizinische Funktion durchzuführen, wobei das Basisteil (124) mindestens eine der Körperoberfläche zuweisende Auflagefläche (142) aufweist, wobei das Basisteil (124) mindestens einen Aufnahmekanal (158) zum Aufnehmen von aus der Insertionsstelle austretender Körperflüssigkeit aufweist.
